# EUROPEAN PATENT SPECIFICATION

(11) **EP 1 781 775 B1**
(45) Date of publication and mention of the grant of the patent: **01.05.2013**
(21) Application number: 04764160.0
(22) Date of filing: 28.07.2004
(51) Int. Cl.: C12N 5/074, A61K 35/44, A61P 9/10

(54) **VASCULAR STEM CELLS AND USES THEREOF**
VASKULÄRE STAMMZELLEN UND DEREN VERWENDUNGEN
CELLULES SOUCHES VASCULAIRES ET LEURS UTILISATIONS

(43) Date of publication of application: 09.05.2007
(73) Proprietor: UNIVERSITE PARIS DESCARTES, 75270 Paris Cedex 06 (FR)
(72) Inventor: SAINZ, Julie, 75016 Paris (FR); AL HAJ ZEN, Ayman, 92130 Issy-les-Moulineaux (FR); LAFONT, Antoine, 75007 Paris (FR)
(74) Representative: Vaillant, Jeanne
(86) International application number: PCT/EP2004/009168
(87) International publication number: WO 2006/010385

(56) References cited:
- WO-A2-03/092471
- US-A1- 2004 072 342
- SAINZ J ET AL: "Identification and characterization of potential stem cells in the vascular wall of normal adult mouse aorta." EUROPEAN HEART JOURNAL, vol. 24, no. Abstract Supplement, August 2003 (2003-08), - September 2003 (2003-09) page 365, XP004530927 & CONGRESS OF THE EUROPEAN SOCIETY OF CARDIOLOGY; VIENNA, AUSTRIA; 30 AUGUST-3 SEPTEMBER 2003 ISSN: 0195-668X
- ZHOU S ET AL: "The ABC transporter Bcrp1/ABCG2 is expressed in a wide variety of stem cells and is a molecular determinant of the side-population phenotype" NATURE MEDICINE, vol. 7, no. 9, September 2001 (2001-09), pages 1028-1034, XP002332849 ISSN: 1078-8956
- TAMAKI T ET AL: "Identification of myogenic-endothelial progenitor cells in the interstitial spaces of skeletal muscle" JOURNAL OF CELL BIOLOGY, vol. 157, no. 4, 13 May 2002 (2002-05-13), pages 571-577, XP002961313 ISSN: 0021-9525
- GOODELL M A ET AL: "Isolation and functional properties of murine hematopoietic stem cells that are replicating in vivo" JOURNAL OF EXPERIMENTAL MEDICINE, vol. 183, no. 4, April 1996 (1996-04), pages 1797-1806, XP000604752 ISSN: 0022-1007 cited in the application
- RAFII S & LYNDEN D: "Therapeutic stem and progenitor cell transplantation for organ vascularization and regeneration." NATURE MEDICINE, vol. 9, no. 6, June 2003 (2003-06), pages 702-712, XP002332851 ISSN: 1078-8956 cited in the application

## Description

### Field of Invention

The present invention relates to a method for obtaining a cell population enriched in vascular stem cells and the vascular stem cells obtained from this method. These vascular stem cells can be used for a variety of purposes, especially in cell therapy for the treatment of ischemic diseases, including peripheral ischemia and myocardial infarction.

### BACKGROUND OF THE INVENTION

Ischemia refers to an insufficient blood supply to any part of the body. The most common cause of ischemia in the heart muscle is due to coronary artery disease, which is a blockage of the arteries preventing oxygen rich blood from reaching the heart. If blood flow in the heart is interrupted, a heart attack occurs.

Patients with advanced coronary artery disease have weakening symptoms such as angina, loss of breath, fatigue, sweating and nausea. In patients with coronary artery disease coupled with severe ischemia some type of intervention must be performed to maintain the life of these patients.

Besides the heart, ischemia can also occur in the skeletal muscles from traumatic injuries to various arteries and is generally known as peripheral ischemia. In this case, there is a possibility of having to amputate a limb if severe ischemia is not properly treated.

Surgical intervention for treating ischemia is a known and practiced method. For example, the treatment of ischemic heart disease with coronary artery bypass surgery or angioplasty can, in most cases, reduce the symptoms of this disease and prolong the life of these patients.

As an alternative to surgical intervention, natural angiogenesis techniques have been sought out. These techniques rely on the body's ability to generate new blood vessels by triggering angiogenesis with growth factors such as FGF-1; VEGF and IGF. In addition, in the case of myocardial infarction, studies using autologous skeletal myoblasts which are transplanted into ischemia-damaged myocardium are being explored.

The problem with the above techniques is that they often result in incomplete revascularization due to the presence of ischemia extending into small peripheral vessels. Furthermore, if the revascularization does occur, it is often too slow a process to completely restore organ function.

Rapid revascularization of injured, ischemic and regenerating organs is essential to restore organ function. Thus, today much scientific effort is being focused on delivering vascular progenitor cells to facilitate restoration of organ revascularization to treat ischemia, as well as other diseases. Several studies have shown that bone marrow-derived cells or cells circulating in the peripheral blood functionally contribute to neoangiogenesis during wound healing and limb ischemia and postmyocardial infarction (see, e.g., Rafii, S., Lynden, D., Nat. Med. 6, 702-712, 2003).

Use of endothelial cell progenitors derived from haematopoietic stem cells for enhancing angiogenesis is described, for example, in US Pat 5,980,887.

Despite enthusiasm for these pioneering clinical trials, it remains to be determined in randomized clinical trials whether these therapeutic strategies will result in longstanding improvement of the revascularization and decrease morbidity and mortality without any long-term toxicity. Late-onset toxicity may emerge as a result of the use of whole populations of bone marrow mononuclear cells, which contain different organ-specific stem, progenitor and haematopoietic cells. This is of critical importance, since the introduction of nonessential cells into organs may be associated with increased toxicity. Since hematopoietic cells can produce, for instance, profibrotic and excessive amounts of angiogenic factors, unnecessary introduction of these cells into mammals may induce formation of edema and dilated hemorrhage-prone vessels. Moreover, if incorporated into regenerating myocardium, these nonessential cells may result in generation of noncardiac or nonvascular tissues and life-threatening arrhythmias. Thus, more complex isolation procedures to obtain pure populations of vascular stem and/or progenitor cells are necessary to avoid future complications and potential clinical setbacks that may halt future trials and decrease the true value of vascular stem-cell therapeutics.

US patent application 20030194802 provides a method for isolating vascular progenitor from embryonic stem cells. However, with a view to clinical applications, using embryonic stem cells poses a variety of ethical and immunological problems, with respect to the supply of cells.

Thus, there is still a need in this art to provide a method for isolating a cell population enriched in vascular stem cells from adult cells.

Therefore, it is an object of the present invention to provide a method for obtaining a cell population enriched in vascular stem cells, which method provides purer vascular stem cells than those known in the art.

It is another object of the present invention to provide a cell population, enriched in vascular stem cells, obtainaide by this method.

Use of the enriched vascular stem cells for the preparation of a cell therapy product to treat diseases associated with ischemia is also an object of the present invention.

A kit containing the cell population the present invention and a biomatrix is also an object of the present invention, as well as a composition containing the vascular stem cells of the present invention and a physiological acceptable carrier.

These and other objects are achieved by the present invention as evidenced by the summary of the invention, the description of the preferred embodiments and the claims.

### SUMMARY OF THE INVENTION

The present invention relates to two populations of arterial cells isolated from mammalian artery: side population cells (SP) and main population (MP) cells. SP cells are also referred to herein as a cell population enriched in vascular stem cells.

The present invention thus provides a method for obtaining a cell population enriched in vascular stem cells comprising a) providing a sample isolated from the medial tunica and internal tunica of a mammalian artery; b) enzymatically digesting the sample of step a) to free vascular cells; c) combining the vascular cells obtained in step (b) with a labelled vital and/or lipophilic substrate for ATP-Binding Cassette Transporters, under conditions appropriate for uptake of the substrate by said cells; d) determining the amount of labelled substrate present in each cell; and e) isolating the population of nucleated cells which contains the lowest amount of substrate, thereby obtaining a cell population enriched in vascular stem cells. In another aspect, the present invention provides a method for obtaining a cell population enriched in vascular stem cells comprising a) providing a sample isolated from the medial tunica and internal tunica of a mammalian artery; b) enzymatically digesting the sample of step a) to free vascular cells; and c) selecting the vascular cells obtained from step b) which express a Bcrp1/ABCG2 transporter.

In yet another aspect, the present invention relates to a method for preparing vascular tissue, comprising culturing a cell population enriched in vascular stem cells according to the invention, in the presence of at least one vasculogenic and/or angiogenic growth factor, under conditions suitable for inducing vascular tissue differentiation, thereby obtaining vascular tissue.

In still another aspect, the present invention provides a method for preparing a vascularized regenerated mammalian tissue, comprising: providing a cell population enriched in vascular stem cells according to the invention; and contacting said cell population with an isolated mammalian tissue under conditions suitable for differentiation of the vascular stem cells into vascular cells, thereby obtaining vascularized regenerated mammalian tissue.

A method for obtaining a cell therapy product is also disclosed. This method comprises providing a cell composition selected from the group of:
a population cell enriched in vascular stem cells according to the invention; a vascular tissue as prepared according to the invention; and a vascularized regenerated tissue as prepared according to the invention; combining an efficient amount of said cell composition with an appropriate vehicle for *in vivo* administration in a mammal, thereby obtaining a cell therapy product.

An *in vitro* method for screening for compounds having angiogenic or anti-angiogenic compound is also disclosed, as well as compositions comprising the cell population enriched in vascular stem cells of the present invention and kits.

### BRIEF DESCRIPTION OF THE DRAWINGS

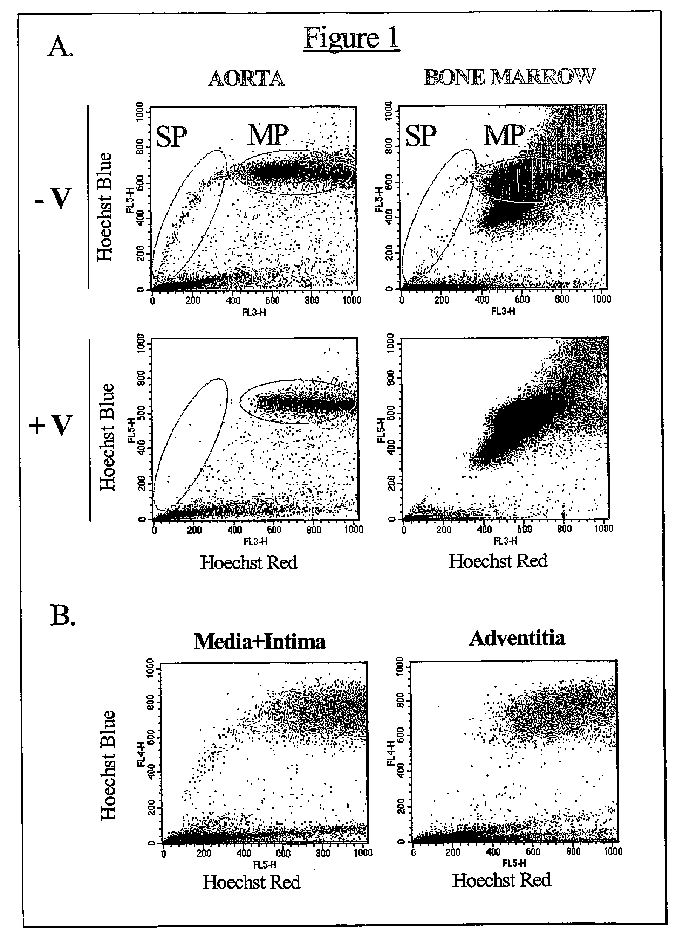

**Fig.1****: *A*. Identification of Vascular Side Population cells (VSP)** and comparison to Bone Marrow SP cells. Shown are Hoechst 33342 and PI staining and emission patterns of aortic cells (left) and bone marrow cells (right) in the absence (top) and presence (bottom) of verapamil (V). Verapamil selectively prevented Hoechst exclusion from SP cells. PI positive dead cells appear far right on the plot. Red blood cells and debris stain negative on the bottom of the plot due to the absence of DNA. B. Hoechst 33342 and PI staining of vascular cells isolated after dissection of the aorta from either layers suggest that VSP cells are localized in the media or intima layer (left) but not in the adventitia (right).

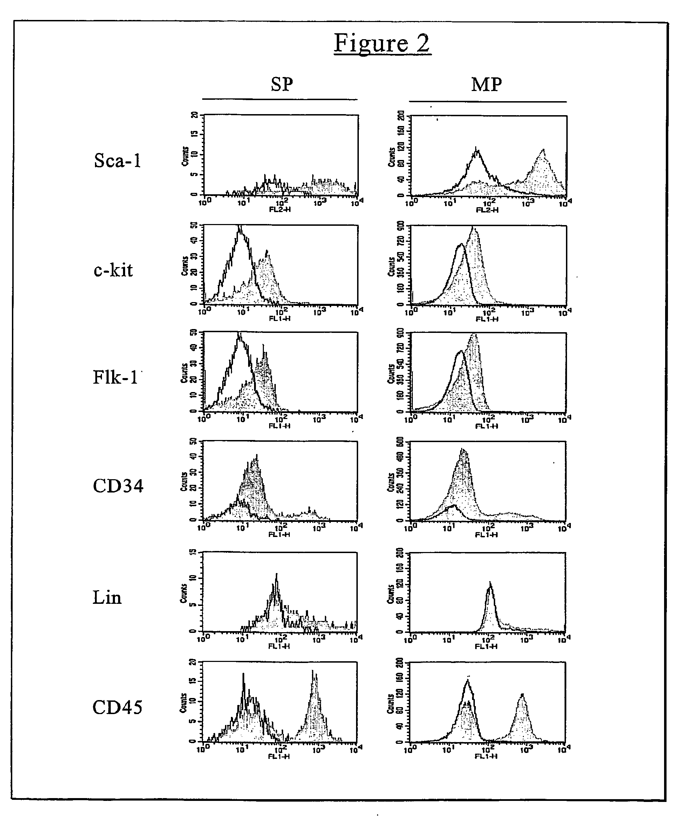

**Fig.2****: Surface Phenotype of Vascular SP** cells. Flow cytometry analysis of vascular cells double labelled with Hoechst 33342 dye and monoclonal antibodies to several stem or progenitor markers. Expression level of these markers was determined for gated SP cells (left) and MP cells (right) and is shown in grey tracing. Control samples where the primary antibody was omitted or replaced by an irrelevant IgG are shown in black solid lines.

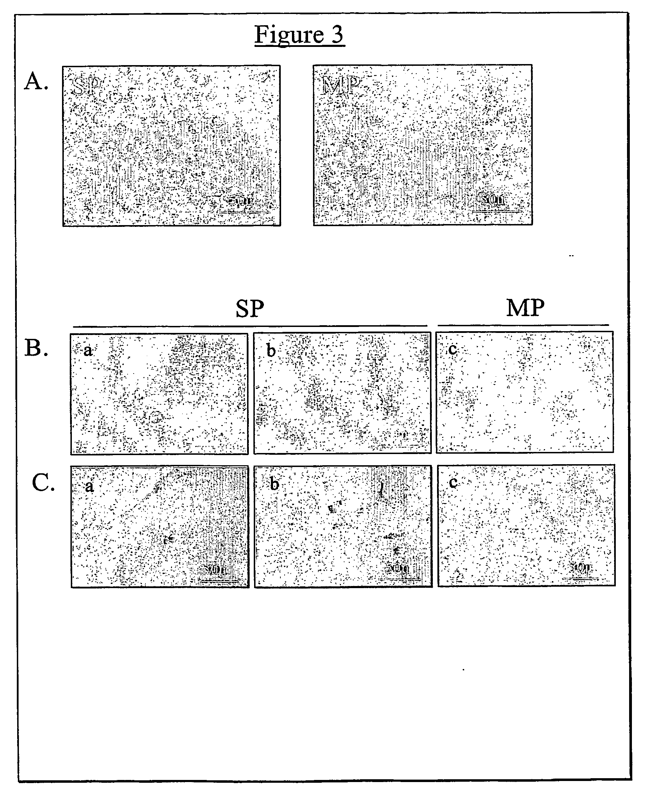

**Fig.3****: Vascular SP and MP cell morphology and Bcrp1 expression. *A*.** SP and MP cells displayed heterogeneous morphology upon microscopic examination prior to culture. ***B*.** Cytospin preparations of Vascular SP cells (a,b) immunostained positively for **Bcrp1** whereas MP cells (c) showed no staining for the marker. C. Vascular SP and MP cells were cultured for 3 days after FACS-sorting and immunostained for Bcrp1. VSP cells confirmed heterogeneous morphology and more contrasted **Bcrp1** expression. VMP cells remained unstained for Bcrp1.

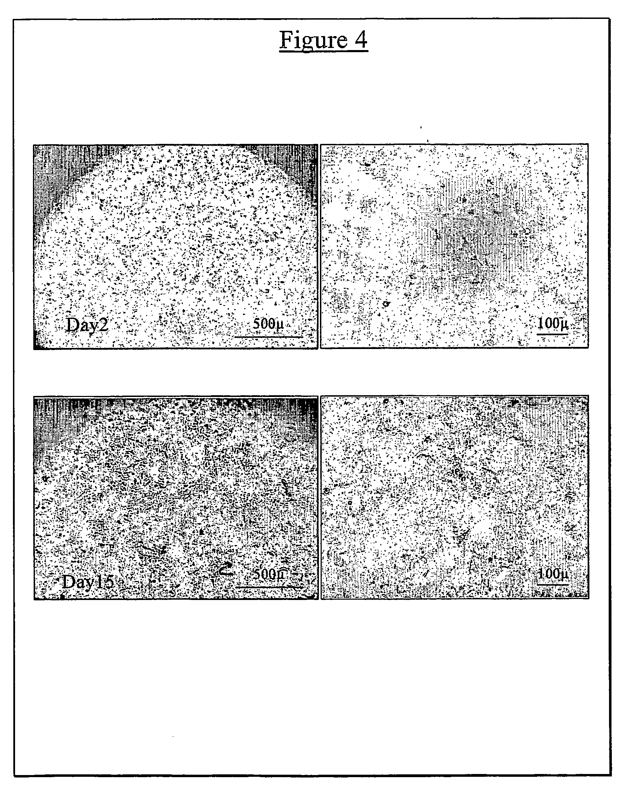

**Fig.4****: Vascular SP cell haematopoietic colony assay.** To determine whether Vascular SP cells had haematopoietic potential, they were cultured in Methocult M3434 for 21 days. Photographs of the assay after 2 and 15 days of culture show their incapacity *in vitro* to form haematopoietic colonies. Right panel photographs represent enlargements of left panel.

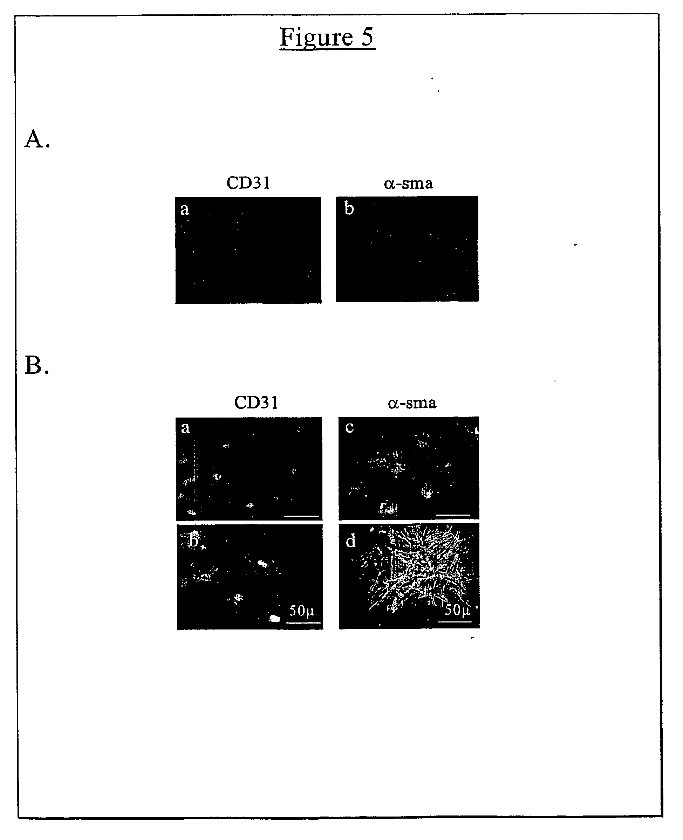

**Fig.5****: Vascular differentiation assay. A.** CD31 and α-sma immunostaining of VSP cell cytospin preparations showed VSP cells expressed none of these vascular markers when immediately extracted from the arterial tissue. ***B*.** After plating and culture in various differentiation conditions, similar immunostainings were performed and illustrated that if some VSP cells remained unstained for the two first markers (a,c), other VSP cells displayed the capacity to differentiate and express the endothelial cell marker CD31 (b) or the smooth muscle cell marker α-sma (d). Hoechst was used for nuclei staining.

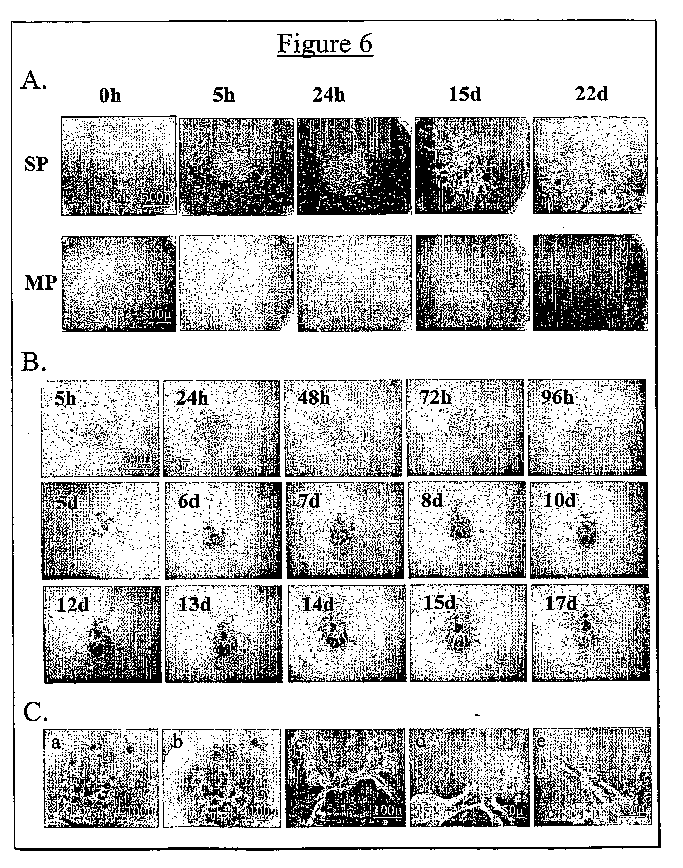

**Fig.6****: Matrigel culture of Vascular SP and MP cells. *A*.** Behaviour of SP and MP cells cultured on matrigel was monitored for 3 weeks. Summarized comparison between the two populations is shown at most relevant points of the time course. While SP cells form vast arboreal vascular-like structures, MP cells do not display such functional properties (h=hours; d=days) B. Detailed kinetic evolution of Vascular SP cells cultured on matrigel. C. VSP cells first aggregate to form spheroids clearly distinguishable 5 days after plating (a) capable of fusing together (b). Black and white * symbols indicate small spheroïds fusing, resulting in the formation of a unique bigger spheroïd designed by identically-colored bigger symbol. Then cells start sprouting from these spheroids to form tube-like structures (d), these structures also being capable of connecting with each others (c).

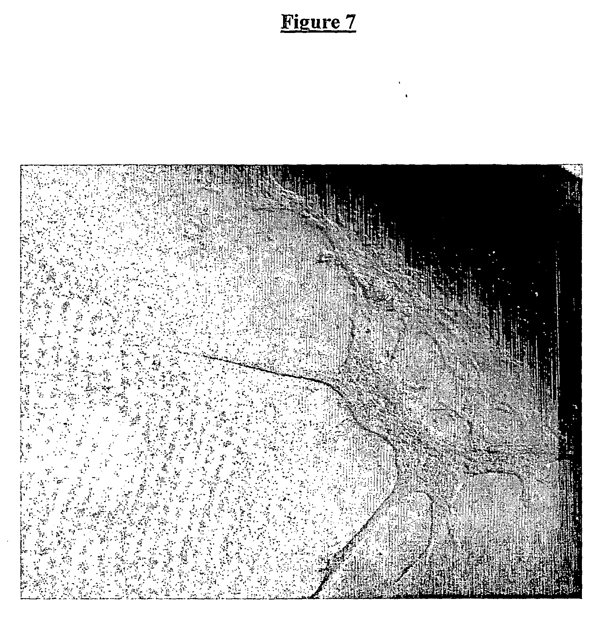

**Fig.7****: Matrigel culture of Vascular SP cells.**

Figure 7 is a photograph showing the matrigel culture of Vascular SP cells.

### DESCRIPTION OF THE PREFERRED EMBODIMENTS OF THE INVENTION

The terms "mammal" and "mammalian", as used herein, refer to any vertebrate animal, including monotremes, marsupials and placentals, that suckle their young and either give birth to living young (eutharian or placental mammals) or egg-laying (metatherian or non-placental mammals). Examples of mammalian species include humans and other primates (e.g., monkeys, chimpanzees), rodents (e.g., rats, mice, guinea pigs) and ruminants (e.g., cows, pigs, horses).

As used herein the term "revascularization" refers to growth of existing blood vessels and/or growth of new blood vessels from stem cells.

As used herein, the terminology "enriched in vascular stem cells" means that the vascular stem cells are in abundance in comparison to other cells.

The word "treat" as used herein encompasses providing medical care to a patient in need thereof or to manage a disease or a condition.

As used herein a "Brcp1 transporter" includes a ABCG2 transporter or a MXR transporter or a ABCP transporter. All of these transporters are encompassed by the present invention since they are referred to in the scientific literature as being the same transporters.

In the method of the present invention a part of an artery is first excised from the mammal and cells are derived therefrom. Any mammalian artery can be used in the method of the present invention including, but not limited to, aortic, femoral, thoracic, abdominal, metacarpal, jugular, radial and the like.

In one aspect, the mammal is a murine and the artery is the aorta. In another aspect, the mammalian is human and the artery removed is the radial artery. In yet another aspect, the mammal is a human patient who suffers from peripheral ischemic disease or who underwent myocardial infarction. The artery is removed following methods that are known in the art such as those described by Reyes et al., Ann Thorac Surg 59:118-26 (1995).

The removed artery can be dissected as follows: surrounding fat and adventitia is gently removed from the artery; the remaining media and intima are carefully minced. For example, one can remove from 2 to 20 cm of the radial artery of a patient. In one aspect, around 10 cm is removed from the patient.

As used herein, the term "adventitia" refers to the external tunica (layer) of the artery, the term "media" refers to the medial tunica (layer) of the artery and the term "intima" refers to the internal tunica (layer) of the artery.

Mincing can be carried out manually with fine scissors.

The minced sample then undergoes enzymatic digestion, for example under rotation in an incubator. As used herein, "to undergo enzymatic digestion" means to be placed in the presence of one or several enzymes which is or are able to dissociate the arterial tissue (extracellular matrix) in order to dissociate the vascular cells.

Appropriate digestive enzymes that are suitable for use alone or in combination in the method of the invention are type II collagenases. Other enzymes may be used, selected among the following enzymes: all the collagenases, the trypsins, dispases or proteases, pronase, elastases; and/or, hyaluronidases.

The amount of enzyme(s) used in the method of the present invention is an amount sufficient to digest the extracellular matrix of the arterial tissue in order to free the vascular cells. The amount of enzyme(s) and the digestion time will vary depending on the particular enzyme or combination of enzymes which are used and on the characteristics of the removed artery.

As an example, for mice, around 430 units of collagenase type II diluted in 1 ml enable suitable digestion of an aorta of a mouse incubated 4 hours at 37°C. In human, incubation time and/or enzyme concentration can be increased.

The digested cells are then re-suspended in an appropriate medium, for example an appropriate culture medium and combined with a labelled substrate for ABC (ATP-Binding Cassette) Transporters according to step (b) of the method. Example of appropriate medium is the cell culture medium DMEM.

As used herein, the term "substrate for ABC (ATP-Binding Cassette) transporters" refers to a substance which is removed from the vascular cell by ABC (ATP Binding Cassette) transporters. According to the invention, a substrate for ABC Transporters is a compound that is not removed from the cells in the presence of an inhibitor of ABC transporters such as verapamil.

The substrate is generally a lipophilic substrate. Substrates for ABC transporters can be selected among the following compounds : topoisomerase inhibitors, and for example anthracyclins and their analogues (mitoxantrone), doxorubicine, daunomycine (epirubicine, idarubicine), epipodophyllotoxines (etoposide VP-16), camptothecines (topotecan, irinotecan, SN-38, exatecan) and indolocarbazoles (NB-506, J-107088), calcheamicine, porphyrines and analogues of porphyrine (pheophorbide, protoporphyrine XI), PhIP, bioflavenoïds, flavopiridol, anti-HIV nucleoside analogues (lamivudine (=3TC), zidovudine (=AZT)), Hoechst 33342, rhodamine 123 and the like.

In a particular embodiment of the method, a substrate of ABC transporters is selected among Hoechst 33342 and rhodamine 123.

Inhibitors of ABC transporters include verapamil and fumitremorgin C (and analogue Ko143). Other inhibitors of ABC transporters can be selected among the inhibitors of **Bcrp1** including vanadate, cyclosporine, reserpine, GF120918, and inhibitors of tyrosine-kinase such as CI1033, gefitinib, imatninb, as well as anti-ABCG2 blocking antibodies such as the monoclonal 5D3 and the like.

For the purpose of the invention, the substrate is labelled. As used herein, the term "labelled" means that the substrate can be easily detected and quantified using an appropriate device. Appropriate labelling includes radiolabelling, enzymatic, chemiluminescent labelling or fluorescent labelling.

In another aspect of the invention, said labelled substrate for ATP-Binding Cassette Transporters is a vital lipophilic fluorescent dye, for example Hoechst 33242. In such embodiment, step d) of the method of the invention is carried out by exposing the cells to an excitation wavelength which causes fluorescence of the dye and by determining fluorescence emission of each cell.

The amount of labelled substrate used in the method of the present invention is an amount sufficient to detect the presence of said substrate into the cells. The amount of labelled substrate will vary depending on the substrate which is utilized and the source of the cells. In another aspect, the amount of fluorescent dye which is used is between about 0.1 µg/ml to 8 µg/ml.

The staining time with the labelled substrate (*i.e*.; the length of time cells are exposed to dye) varies depending on the temperature at which staining is to occur and the dye concentration used. In another aspect, the staining time with a fluorescent dye is about 60 to 120 minutes, for example 90 minutes.

The temperature at which staining with the dye can be carried out is generally from about 35° C to about 40°C but may also vary about 37°C.

In another aspect of the method, the cell populations are combined with 5 µg/ml of Hoechst 33342 for 60 to 120 minutes, for example, 90 minutes at 37°C.

The resulting combination is exposed to an excitation wavelength which results in fluorescence of the dye, which is observed at an emission wavelength. The amount of dye contained in each cell type resolved at the emission wavelength is determined. As it is reported hereafter, it has been found that the population of nucleated cells which contains the lowest amount of dye at the emission wavelength, relative to the other population of nucleated cells, is a cell population enriched in vascular stem cells. Therefore, the population of nucleated cells which contains the lowest amount of dye at the emission wavelength, relative to the other population of nucleated cells, is isolated.

In another aspect, when using Hoechst 33342 dye, said excitation wavelength is about 351 nm and said emission wavelength is selected from 400 to 700 nm depending upon each cell type.

The cell population which contains the lowest amount of labelled substrate is isolated using the various methods available in the art to carry out selective sorting procedure. As an example, one can use the techniques of sorting by cloning, by flow cytofluorimetry, by immunoaffinity or immunomagnetic columns using specific antibodies and the like.

A sorting procedure that can be used in the method of the present invention is a FACS sorting procedure, which can be carried out when using a fluorescent labelled substrate, such as Hoechst 33342 (Sigma Aldrich Chimie SARL, Saint Quentin Fallavier, France).

The intensity of fluorescence of a population of nucleated vascular cells in an artery sample (i.e., corresponding to the amount of labelled substrate present in a population of nucleated vascular cells), relative to the other population of nucleated vascular cells in the artery sample, is determined by comparing the intensity of fluorescence between each cell population. This information is used to define the area in the sorting profile where the vascular stem cells reside.

FACS sorting is thus a convenient method to determine and isolate the population of nucleated cells which contains the lowest amount of dye at the emission wavelength relative to the other population of nucleated cells, resulting in obtaining a cell population enriched in vascular stem cells.

In yet another aspect, a dye which does not affect the staining profile of the fluorescent vital dye but which allows exclusion of dead cells (e.g. Propidium Iodide) is added in addition to the fluorescent vital dye.

Typically, as a negative control, vascular cells isolated from mammalian artery are stained with a fluorescent vital dye in the presence of an inhibitor of ABC transporters (e.g. Verapamil, Sigma Aldrich). Simultaneously, as the test sample, a second sample of vascular cells is stained with the fluorescent vital dye in the absence of the inhibitor. The stained samples (negative control and test sample) are exposed to an excitation wavelength which results in fluorescence of the dye, which is determined at an emission wavelength. The cell populations observed in the negative control are compared with the cell populations observed in the test sample. Vascular stem cells will be visible in the test sample but will not be visible in the negative control. This approach can be used to define the location (set the gate) for isolation of vascular stem cells using the methods of the present invention.

Fluorescence intensity can be determined at one emission wavelength. Alternatively, fluorescence intensity can be determined at two emission wavelengths. Suitable emission wavelengths are those which will measure fluorescence of the dye used in the method of the present invention so that distinct populations of live vascular cells are resolved as a result of the differences in intensity of fluorescence. For example, Hoechst 33342 emission can be detected at a range of wavelengths of about 450nm to about 700nm, and in one aspect, about 670nm. Hoechst 33342 emission can also be detected at simultaneous wavelengths of about 424nm and about 670nm. Hoechst 33342 emission is detected with a 424nm long pass filter. Propidium iodide fluorescence can be detected with a 670nm long pass filter.

The composition may be further enriched for vascular stem cells by positive selection or for known stem cell specific markers and/or negative selection for lineage specific markers, or by size selection using filtration.

One marker specific of the cell population enriched in vascular stem cells according to the invention is the **Bcrp1** transporters. Accordingly, the application reports the use of a compound which binds specifically to Bcrp1 transporter for detecting and/or purifying vascular stem cells from mammalian artery cells. In another aspect, the compound which binds specifically to **Bcrp1** transporters is selected among the antibodies which are raised against Bcrp1. Polyclonal or monoclonal antibodies raised against **Bcrp1** transporters can be obtained according to methods well known in the art, for instance by following the procedures exemplified in Sambrook et al, Molecular Cloning, A Laboratory Manual (January 2001), incorporated herein by reference.

The invention thus provides a method for obtaining a cell population enriched in vascular stem cells, comprising
a) providing a sample isolated from the medial tunica and internal tunica of a mammalian artery;
b) enzymatically digesting the sample of step a) to free vascular cells; and
c) selecting the vascular cells obtained from step b) which express a Bcrp1/ABCG2 transporter_{.}

In one aspect, step c) is carried out by FACS sorting. As previously disclosed, steps for enriching or depleting the cell population of a particular cell type can be further carried out.

Various techniques may be employed to carry out positive selection. Monoclonal antibodies are particularly useful to identify markers associated with particular cell lineages and/or stages of differentiation. Techniques for positive selection or separation may include, but are not limited to, magnetic separation, using antibody-coated magnetic beads, affinity chromatography and panning with antibody attached to a solid matrix, e.g., plate, elutriation.

The cell populations of the invention may be modified by appropriate gene transfer, recombination, to correct genetic defects or provide genetic capabilities naturally lacking in the stem cells or their progeny. For example, the cells can be genetically engineered to be used as a vector for a gene.

The present invention thus provides a cell population enriched in vascular stem cells obtainable by the methods of the invention as here-above defined. The application also describes a cell population enriched in vascular stem cells and isolated from mammalian artery, comprising between 83.6% to 91.6% Sca-1+ cells, between 49% to 49.8% c-kit+ cells, between 53% to 57.6% Flk-1+ cells, between 8.2% to 9.4% CD34+ cells and between 67.4% and 78.8% Lin- cells.

In one aspect the cell population enriched in vascular stem cells according to the invention is a human cell population.

Characterization of the murine cell population according to the present invention revealed them to be able to differentiate *in vitro* in conditioned medium in both endothelial and smooth muscle cells, which are the two main cell types that constitute the arterial wall tissue. As described hereafter, it has now been surprisingly found that, the cell populations of the present invention are able to form vascular-like structures, especially when cultured *in vitro* on a 3D matrix support.

Thus, the invention also provides a method for preparing vascular tissue. The method is carried out by culturing the cell population enriched in vascular stem cells according to the invention in the presence of at least one vasculogenic and/or angiogenic growth factor, under conditions suitable for inducing vascular tissue differentiation, optionally followed by one or more expansion phases.

In one aspect, vascular tissue is prepared by culturing the cells in a semi-solid vascularization-promoting medium. Such a medium typically comprises extracellular matrix components (for example Matrigel™ basement membrane matrix (Discovery Labware, Beckton Dickinson) or any convenient polymeric 3D scaffold. Appropriate growth factor may be any of known endothelial cell mitogen. Such endothelial cell mitogen include, for example, acidic and basic fibroblast growth factors (aFGFand bFGF), vascular endothelial growth factor (VEGF), angiopoietin (Ang), ephrin (Eph), Placental growth factor (PIGF), epidermal growth factor (EGF), transforming growth factor α and β (TGF-α and TGF-β), platelet-derived endothelial growth factor (PDEGF), platelet-derived growth factor (PDGF), tumor necrosis factor α (TNF-α), hepatocyte growth factor (HGF), insulin growth factor (IGF), erythropoietin, colony-stimulating factor (CSF), macrophage-CSF (M-CSF), granulocyte/macrophage CSF (GM-CSF) and nitric oxidesynthase (NOS)

Examples of growth factors that can be used in a matrix comprising laminin, collagen IV and entactin, are EGF, bFGF, NGF, PDGF, IGF-1 and TGF-β. For example, the matrix comprises laminin (56%), collagen IV (31%) and entactin (8%), EGF (0.5-1.3ng/ml), bFGF (<0.1-0.2pg/ml), NGF (<0.2ng/ml), PDGF (5-48pg/ml), IGF-1 (11-24ng/ml), and TGF-β (1.7-4.7ng/ml).

In another aspect, the medium used for growth and differentiation into vascular tissue is a medium comprising fetal bovine serum, hydrocortisone, hFGF-B, VEGF, R³-IGF-1, ascorbic acid, hEGF, GA-1000 and heparin, such as EGM™-2-endothelial cell medium-2 as commercialised by Cambrex Bioscience.

Again, the method may comprise the steps of depleting or selecting or inhibiting the growth of one or several specific cell types among the cell types present in the cell population provided in step (a).

In addition to the above application of the cell population of the invention, cell population enriched in vascular stem cells can be used to provide vascularization of non-vascular, or inherently poor vascularized tissue, especially, to provide vascularization of tissue engineered *in vitro* for implantation. Thus, the invention provides a method for preparing a vascularized regenerated mammalian tissue. The method is carried out by
(a) providing a cell population enriched in vascular stem cells according to the invention,
(b) contacting the cells with an isolated mammalian tissue under conditions suitable for differentiation of the vascular stem cells into vascular cells.

As used herein, an "isolated mammalian tissue" refers, either to removed tissue from a mammalian or to an engineered tissue derived from *in vitro* cell cultures prepared for implantation. Examples of such engineered tissue are masses of *in vitro* prepared cells selected among hepatocytes, epidermal and dermal cells, pancreatic, skeletal muscle tissue, smooth muscle tissue such as urinary bladder smooth muscle tissue, myocardiac tissue, adipose, cartilaginous and bone tissue, prepared for implantation. Contacting the mammalian tissue with the cells can be carried out by co-culture in semisolid matrix or on a porous scaffold.

The cell population enriched in vascular stem cells, the living vascular tissue or the vascularized regenerated tissue as prepared by the methods of the invention, can be used for cell therapy and/or for *in vivo* neovascularization of non-vascular tissue.

As used herein, the term "cell therapy" refers to a method for reconstituting damaged tissue or for restoring a biological function that has been lost or impaired within a tissue, comprising transplanting cells that have been prepared ex *vivo* onto the sick tissue.

The present invention thus provides a method for obtaining a cell therapy product comprising
a) providing a cell composition selected from the group of:
   a. a cell population enriched in vascular stem cells according to the invention;
   b. a vascular tissue as prepared according to the invention; and
   c. a vascularized regenerated tissue as prepared according to the invention,
b) combining an efficient amount of said cell composition with an appropriate vehicle for *in vivo* administration in a mammal,
thereby obtaining a cell therapy product.

An "efficient amount of cell composition" is defined herein as the amount of cell composition which, when administered to a mammal, is sufficient for therapeutic efficacy (e.g., results in clinical improvement) (e.g., an amount sufficient for treating or eliminating symptoms and/or signs associated with the disease of interest).

For example, in the case of a mammal with peripheral arterial disease or myocardial infarction, an effective amount of a cell population enriched in vascular stem cell is that amount of cells, which when administered to the mammal, can revascularize the ischemic tissue. From about 10⁴ to 10¹⁰ cells may be administered to the patient for transplantation.

The cell therapy product described herein can be used for treating, in a mammal, ischemia, or cardiovascular disease. The cell therapy product obtained by the above method can be used to enhance blood vessel formation in ischemic tissue. Such tissue can include for example, muscle, brain, kidney and lung. Ischemic diseases include, for example, cerebrovascular ischemia, renal ischemia, pulmonary ischemia, limb ischemia, ischemic cardiomyopathy and myocardial ischemia.

In another aspect, the cell therapy product is used for the treatment of peripheral vascular ischemia.

Some patient populations, typically elderly patients, may have either a limited number of endothelial progenitor cells or a limited number of functional endothelial progenitor cells. Thus, if one wants to promote angiogenesis, for example, to stimulate vascularization by using a potent angiogenesis promoter such as VEGF, it is advantageous to provide a cell therapy product discribed herein in combination with said angiogenesis treatment. Thus, the the cell therapy product described herein is used to potentiate a patient for angiogenesis in combination with a potent angiogenesis promoter, such as VEGF.

The invention further concerns use of the cell composition according to the invention, for the preparation of a cell therapy product intended for the treatment of ischemia or cardiovascular disease.

For example, when the mammal is a human suffering from post-ischemic cardiac failure or cardiovascular disease, the cell population enriched in vascular stem cells is isolated from an artery of the human suffering from post-ischemic cardiac failure or cardiovascular disease.

The cell therapy product as described herein can be Implanted into embryonic, growing or adult organisms suffering from insufficient or faulty vascularization, as in the microvascular pathology of diabetes, or into tissues having or at risk for ischemic damage, as in ischemic heart disease and cerebral-vascular disease. Similarly, cell populations enriched in vascular stem cells or vascular tissue of the present invention can provide blood vessels of large diameter for tissue replacement therapy in cases of surgical bypass, vascular degeneration such as atherosclerosis and autoimmune disease.

Additionally, injuries from vascular trauma due to devascularized skeletal muscles can also be treated with the cell therapy product described herein to avoid limb amputation.

The cell therapy product as described throughout can be used immediately or frozen in liquid nitrogen and stored for long periods of time, being thawed and capable of being reused. The cells are typically stored for a long period in 10% DMSO.

The present application also describes a method for transplanting an effective amount of a donor cell therapy product into a mammal in need of such treatment (also referred to as a recipient or a recipient mammal).

As used herein, "donor" refers to a mammal that is the natural source of the cell populations of the invention. In a particular embodiment, the donor and the recipient are matched for immunocompatibility. Preferably, the donor and recipient are matched for their compatibility for the major histocompatibility complex (MHC) (human leukocyte antigen (HLA))-class I (e.g., loci A,B,C) and -class II (e.g., loci DR,DQ,DRW) antigens. Immunocompatibility between donor and recipient are determined according to methods generally known in the art (see, e.g., Charron D.J., Curr. Opin. Hematol., 3:416-22 (1996); Goldman J., Curr. Opin. Hematol., 5:417-18 (1998)). In another aspect, the recipient is a human patient. The cell therapy product can be prepared from cells of a patient's healthy artery and be returned, after being isolated and expanded, to the same patient in skeletal muscle or in myocardium that displays peripheral ischemic disease or that underwent infarction, respectively.

The amount of donor cell therapy product administered to a mammal, including frequency of administration, will vary depending upon a variety of factors; including mode and route of administration; size, age, sex, health, body weight and diet of the recipient; the disease or disorder being treated; the nature and extent of symptoms of the disease or disorder being treated; kind of concurrent treatment, frequency of treatment, and the effect desired.

In another aspect of the invention, the invention relates to the use of a cell population enriched in vascular stem cells or a vascular tissue according to the invention, for *in vitro* screening of angiogenic or anti-angiogenic compounds.

More specifically, the invention provides an *in vitro* method for screening of compounds having angiogenic or anti-angiogenic compound, said method comprising:
a) providing a cell population enriched in vascular stem cells or a vascular tissue according to the invention;
b) combining said cell population or said vascular tissue with a candidate compound;
c) culturing said cell population or said vascular tissue under conditions appropriate for growth and differentiation into vascular cells;
d) determining whether the differentiation of vascular stem cells into vascular cell is enhanced or inhibited, thereby indicating that said candidate compound is respectively an angiogenic or an anti-angiogenic compound respectively.

In yet another aspect the present invention provides a composition comprising:
a) enriched vascular stem cells of the present invention; and
b) a physiologically acceptable carrier.

Any physiologically acceptable carrier that is compatible with the enriched vascular stem cells can be used in the composition of the present invention, including, but not limited to saline and the like. For example, a physiologically acceptable carrier can be selected among the group consisting of Phosphate Buffer Saline, NaCl 0.9%, autologous serum and cell culture medium suitable for human administration.

This composition can be used to revascularize ischemic or injured muscle cells, both skeletal and myocardial.

In yet another aspect, the present invention provides a kit containing the enriched population of vascular stem cells, reagents necessary for their culture and a optionally a support matrix.

In order to further illustrate the present invention and advantages thereof, the following specific examples are given, it being understood that the same are intended as illustrative and in nowise limitative.

### EXAMPLES

The following methods were used in examples 1-6.

### Mouse Strains

C57BI/6 female mice from 5 to 8 weeks of age were used for SP isolation and characterization. All C57BI/6 mice were purchased from Charles River France Laboratories (Les Oncins ,France) and the Centre d'Elevage Roger Janvier (Le-Genest-Saint-Isle, France).

All animals were maintained at the Necker Medical School animal facility according to institutional guidelines.

### Isolation of Vascular Cells

Anesthesized C57BI/6 mice were perfused with a solution of HBSS1X, 2%SVF, 10mM Hepes. Thoracic and abdominal aortas were removed and dissected as following. Surrounding fat and adventitia were gently removed under Leica MZ6 stereomicroscope (Leica Microsystems SA, Rueil-Malmaison, France), the remaining Media and Intima were carefully minced and underwent enzymatic digestion during 4 hours with type II collagenase (Worthington Biochemical Corporation, Lakewood, NJ, USA) under rotation in an incubator at 5% CO2, 37°C. 430 enzymatic units were dissolved in 1 ml of D-MEM for digestion of one murine aorta. The digestate was passed through a 100µm nylon filter and resuspended in fresh medium to stop enzymatic reaction.

### Hoechst, Verapamil and Antibody Staining for FACS Analysis

Hoechst staining was performed as follows. Cells were resuspended at 1×10⁶ cells/ml in Dulbecco's Modified Eagle's Medium (D-MEM) with high glucose (Invitrogen, Cergy Pontoise, France) and incubated with Hoechst 33342 (5µg/ml; Sigma Aldrich Chimie SARL, Saint Quentin Fallavier, France) with or without 100µM verapamil (Sigma Aldrich) at 37°C for 90 min. Immunostaining was then carried out at 4°C by using antibodies reactive to Sca-1, Lineage Cocktail, CD45, c-kit, Flk-1 or CD34 conjugated with fluorescein isothiocyanate (FITC) or phycoerythrin (PE) (Pharmingen, Beckton Dickinson Biosciences, Le Pont de Claix, France). Flow cytometry analysis was carried out on a BD™ LSR (Immunocytometry Systems, Beckton Dickinson Biosciences) and accompanying CELLQuest^{R} software (San Jose, CA, USA). Hoechst dye was excited at 351 nm by UV laser and its fluorescence was detected at two wavelengths using 424/44 (Hoechst Blue) and 670/LP (Hoechst Red) filters. FITC and PE were excited at 488 nm by argon laser and their fluorescence detected at FL-1 (530/40) or FL-2 (580/30) filter, respectively. Dead cells and debris were excluded from the plots based on propidium iodide (PI) staining (2µg/ml; Sigma Aldrich).

### Hematopoietic Colony Assay

After isolation, vascular cells were stained with Hoechst and PI. A cell population enriched in vascular stem cells was sorted using a BD™ FACSVantage (Beckton Dickinson) and immediately cultured on 96-well plates in Methocult M3434 Medium (StemCell Technologies, Meylan, France). Haematopoietic colony formation and cell morphology were monitored with a Leica DM IL Microscope and Leica DC300F digital camera.

### Bcrp1 immunostaining of VSP cells

**Bcrp1** immunostaining was performed on sorted cells collected onto glass slides by cytospin, by using a Bcrp1 rabbit polyclonal antibody (1: 100; Kamiya Biomedical, Seattle, WA USA) followed by an HRP-conjugated goat anti-rabbit Ig antibody (1 :100; P0448; DAKOCytomation, Trappes, France). Immunostaining was revealed with liquid diaminobenzidin (DAB) (DAKOCytomation). Prior to staining, endogenous peroxidase activity was neutralized by a peroxidase-blocking reagent (DAKOCytomation) and background staining was avoided by incubation with 10% goat serum in PBS1X as a blocking solution.

### SMC and EC Differentiation Assay

Cells were FACS-sorted and either immediately submitted to cytospin or cultured on 96-well plates in either EGM™-2-endothelial cell medium-2 (CAMBREX Bioscience Paris SARL, Emerainville, France) for endothelial differentiation assays or D-MEM high glucose. After 10 days of culture or consecutively to cytospin, cells were fixed with 4% paraformaldehyde, permeabilized with 0.2% TritonX100, and immunostained with either mouse anti-a-sma (1:50 ;clone 1A4; Oncogene™ Research Products, San Diego, CA, USA) or rat anti-CD31 (1:50; clone MEC13.3; Pharmingen) antibodies. Cells were then incubated with either biotinylated rabbit anti-mouse Ig (1: 100; E0354; DAKOCytomation) or biotinylated rabbit anti-rat Ig (1:50; E0468; DAKOCytomation), and finally AlexaFluor 546-conjugated Streptavidin (1:200 ;Molecular Probes Europe BV, Leiden, The Netherlands) or AlexaFluor 488-conjugated Streptavidin (1:100; Molecular Probes). Prior to staining, endogenous biotin was neutralized by a biotin-blocking system (DAKOCytomation) and background staining was prevented by incubation with 10% goat serum in PBS1X as a blocking solution. Three independent experiments were performed. Immunofluorescence was observed on a Leica DM IL Microscope with a Leica DC300F digital camera and Leica IM50 Image Manager V1.20 Software.

### Fibroblast Differentiation Assay

Whole aortas from C57BI/6 mice were dissected as follows. Adventitia was removed from mice aortas and cut in 2 mm² pieces cultured in 96-well poly-D-Lysine-coated plates (Discovery Labware, Beckton Dickinson) with D-MEM high glucose supplemented with 20% fetal calf serum (FCS) (Invitrogen) in order to obtain fibroblasts. About 3000 FACS-sorted GFP⁺ SP cells were co-cultured with cells originating from 7 day adventitia explant cultures. Co-cultures were maintained in Methocult M3434 for 3 days and in 75% of the same media with 25% of D-MEM high glucose for 8 additional days. After 11 days of co-culture, immunostaining was performed on 4% paraformaldehyde-fixed cells. Incubation with mouse anti-vimentin (1:50; clone RV202; Pharmingen) antibody was first carried out, followed by incubation with biotinylated rabbit anti-mouse Ig (1: 50; DAKOCytomation) and finally AlexaFluor 546-conjugated Streptavidin (1:200; Molecular Probes). Differentiation of GFP⁺ SP cells into fibroblasts after co-culture was evaluated by defining vimentin positive cells co-expressing GFP. Background staining was blocked as mentioned before. Three independent experiments were performed. Immunofluorescence was observed as previously.

### 3D Matrigel Culture

7000 FACS-sorted VSP or VMP cells were cultured in single well on undiluted Matrigel™ basement membrane matrix (Discovery Labware, Beckton Dickinson) in EGM™-2-endothelial cell medium-2 (CAMBREX Bioscience) on 96-well plates. The development of 3D vascular-like structures was monitored for 3 weeks.

### Example 1

### Identification of Vascular Side Population cells (VSP cells) and Vascular Main Population cells (VMP cells) in the adult vascular wall (Fig.1)

Since bone marrow SP cells are the best characterized, the results previously described by Goodell *et al.* (Goodell M.A. et al, J. Exp. Med., 183:1797-1806 (1996)) on the bone marrow side population were first reproduced. Bone marrow cells were collected from femurs and tibiae of C57BI/6 mice and stained with Hoechst. A similar Hoechst staining pattern was observed. To confirm that the fluorescence pattern was specific for the Hoechst staining of the SP cells, the cell suspension was incubated with verapamil which is, beside a well-known calcium channel inhibitor, an inhibitor of the ABC Transporters. The addition of verapamil led indeed to an inhibition of the Hoechst efflux by the SP cells, the Hoechst accumulating similarly in the main and the side population and conducting to the disappearance of the Hoechst-low profile of the SP cells, whereas MP cells remained unaffected.

Secondly, the existence of vascular stem cells aimed to identify their localization in the adult aorta wall was investigated. To this aim, adventitia from C57BI/6 mice aortas was carefully removed and both adventitia and the remaining media and intima were digested by collagenase, the resulting cell suspension being stained with Hoechst. Very few cells from the adventitia were found to expel the dye, whereas 6% of the media and intima living cells displayed an SP profile towards Hoechst staining. These results indicated the identification of a population of cells from the artery (herein referred to vascular SP cells).

### Example 2

### Flow Cytometry characterization of the VSP cells and VMP cells (Fig.2)

To further characterize the SP cells, their expression of several cell surface markers commonly used to identify stem or progenitor cells by immunostaining experiments revealed by flow cytometry was evaluated. Immunophenotyping revealed that among SP cells, there were: between 83.6% to 91.6% Sca-1+ cells, between 49% to 49.8% c-kit+ cells, between 53% to 57.6% Flk-1+ cells, between 8.2% to 9.4% CD34+cells, between 67.4% to 78.8% Lin- cells and between 39.2% to 43.6% CD45- cells. These results show that most VSP cells possess several stem cell markers, whereas few express CD34, a rather downstream progenitor marker. Interestingly, at least 40% of the VSP cells did not express CD45 which is a bone-marrow derived cell marker. Compared to SP cells, MP cells displayed a slightly different expression pattern of these surface markers. The results of the FACS analysis are shown in Table 1 below.

**Table 1**

| Selected Markers | % SP | % MP |
|---|---|---|
| CD34+ | 8.8%± 0.6 | 14.0% ± 3.9 |
| CD45- | 41.4% ± 2.2 | 47.0% ± 0.4 |
| c-kit+ | 49.4% ± 0.4 | 37.0% ± 0.9 |
| Flk-1+ | 55.3% ± 2.3 | 40.9% ± 5.9 |
| Sca-1 + | 87.6% ± 4.0 | 72.7% ± 1.4 |
| Lin- | 73.1 % ± 5.7 | 49.6% ± 0.3 |

### Example 3

### VSP cells share heterogeneous morphology but all express the ABC-Transporter Bcrp1 (Fig.3)

Since none of the surface markers described above appeared clearly relevant to identify specifically VSP cells, the finding of an accurate marker was undertaken. Zhou *et al* workers showed that if several ABC Transporters were expressed in SP cells, only the ABC Transporter Bcrp1 was associated with the Hoechst efflux properties necessary for the SP phenotype. Moreover, considering that verapamil successfully inhibited the Hoechst expel of the SP cells, it was hypothesized that aorta SP cells expressed the **Bcrp1** transporter. Therefore its expression on cultured VSP and VMP cells isolated by FACS sorting was assessed. Immunocytochemistry experiments revealed that when directly extracted from the aortic wall (*i.e*., without undergoing culture), all VSP cells expressed Bcrp1. However, these cells displayed heterogeneous morphology size: small and big cells compose the Side Population. After plated in culture, the small cells remained round but surely adherent, whereas the larger cells spread on the culture dish. No **Bcrp1** staining was found on VMP cell surface, revealing the absence of the transporter on the rest of the vascular cells. Hence **Bcrp1** was an appropriate marker to identify the VSP cells.

### Example 4

### VSP cells do not exhibit hematopoietic potential (Fig.4)

To elucidate whether VSP cells could differentiate into hematopoietic cells *in vitro,* these cells were cultured in methylcellulose medium containing stem cell factor, several interleukins and erythropoietin enabling the growth of myeloid-type hematopoietic cells. After 3 weeks of culture, no colonies were detected, indicating that SP cells from the adult aorta do not display haematopoietic potential for the myeloid lineage. Besides, VSP cells interestingly went from a heterogeneous phenotype described above to a rather epithelioïd phenotype when grown to confluence.

### Example 5

### VSP cells can undergo differentiation into endothelial and smooth muscle cells (Fig.5)

The expression of vascular markers on VSP cells freshly extracted from the aorta was first evaluated. Cells were not plated prior to staining in order not to introduce any artefact that could modify their expression pattern. VSP cells were negative for CD31 and α-sma when directly stained after the enzymatic digestion of the aorta. In order to assess the capacity of the VSP cells to give rise to differentiated endothelial and smooth muscle cells, VSP cells were cultured in appropriate medium respectively. After 10 days of culture, part of VSP cells were able to express α-sma and CD31. In conclusion, VSP cells were capable of differentiating into endothelial and smooth muscle cells.

### Example 6

### VSP cells form 3D vascular-like structures in Matrigel (Fig.6 and 7)

In order to further investigate the capacities of the VSP cells, these cells were cultured on matrigel. This initial aim was to confirm that the VSP cells had endothelial differentiation potential. Surprisingly, VSP cells created very complex structures. VMP cells did not give rise to any structure at all. Within 24 hours to 4 days, VSP cells first aggregated to form spheroïds. Rapidly, a first set of cells started sprouting out of the spheroïds building tubular structures resembling the framework of a vessel. Then a second set of cells migrated longitudinally from the spheroids on the pre-existing tube-like structure. This sprouting occurred at the same time from all spheroids, some of the spheroids even establishing connections between them via the junction of their respective tubes. It is also to be noticed that after connecting to each other some of the spheroids even closed and finally fused together. Moreover, many of the nascent tubes emerging from the spheroids divided at their end into two similar tubes that divided themselves at their end to form two other tubes, the whole process resulting *in fine* in a vast arboreal structure radiating from each spheroid. In 3 weeks, 7000 VSP cells by a process resembling vasculogenesis succeeded to build a complex and wide arboreal structure able to totally invade the underlying matrigel and to spread on the whole surface of the well of the 96-well plate where they were seeded.

## Claims

1. A method for obtaining a cell population enriched in vascular stem cells comprising:
a) providing a sample isolated from the medial tunica and internal tunica of a mammalian artery;
b) enzymatically digesting the sample of step a) to free vascular cells;
c) combining the vascular cells obtained in step (b) with a labelled vital and/or lipophilic substrate for ATP-Binding Cassette Transporters, under conditions appropriate for uptake of the substrate by said cells;
d) determining the amount of labelled substrate present in each cell; and
e) isolating the population of nucleated cells which contains the lowest amount of substrate, thereby obtaining a cell population enriched in vascular stem cells.

2. The method of claim 1, wherein said labelled substrate for ATP-Binding Cassette Transporters is a vital fluorescent dye.

3. The method of claim 2, wherein said step d) is carried out by exposing the cells to an excitation wavelength which causes fluorescence of the dye and determining fluorescence emission of each cell.

4. The method of claim 3, wherein said dye is Hoechst 33342 dye, said excitation wavelength is 351 nm and an emission wavelength from 400 to 700 nm is determined for each cell.

5. The method of any one of claims 2 to 4, wherein steps d) and e) are carried out by using a FACS device.

6. The method according to any one of claims 1 to 5, wherein a dye which allows exclusion of dead cells is added in addition to the labelled substrate.

7. The method of claim 6, wherein said dye allowing exclusion of dead cells is propidium iodide.

8. The method according to any one of claims 1 to 7, wherein said cells provided in step a) are isolated from a human artery.

9. The method according to any one of claims 1 to 8, wherein said sample is digested with type II collagenase(s).

10. Use of an antibody raised against Bcrp1/ABCG2 for detecting and/or purifying vascular stem cells from mammalian artery cells.

11. The use according to claim 10, wherein said antibody is a polyclonal antibody raised against a Bcrp1/ABCG2 transporter.

12. The use according to claim 10, wherein said antibody is a monoclonal antibody raised against a Bcrp1/ABCG2 transporter.

13. A method for obtaining a cell population enriched in vascular stem cells, comprising:
a) providing a sample isolated from the medial tunica and internal tunica of a mammalian artery;
b) enzymatically digesting the sample of step a) to free vascular cells; and
c) selecting the vascular cells obtained from step b) which express a Bcrp1/ABCG2 transporter.

14. The method of claim 13, wherein step c) is carried out by FACS sorting.

15. A cell population enriched in vascular stem cells obtainable by the method of any one of claims 1 to 9.

16. A cell population according to claim 15 that is a human cell population enriched in vascular stem cells.

17. A cell population enriched in vascular stem cells, **characterized in that** it is obtainable by the method of claims 13 or 14.

18. A method for preparing vascular tissue, comprising culturing a cell population enriched in vascular stem cells according to any one of claims 15 to 17, in the presence of at least one vasculogenic and/or angiogenic growth factor, under conditions suitable for inducing vascular tissue differentiation, thereby obtaining vascular tissue.

19. The method according to claim 18, further comprising one or more expansion phases after differentiation of said vascular tissue.

20. The method of claim 18, wherein said culture step is carried out in a semi-solid vascularization-promoting medium comprising extracellular matrix components.

21. The method of claim 20, wherein said extracellular matrix component is a **Matrigel** basement membrane or collagen gel.

22. The method of claim 20 or 21, wherein said medium comprises VEGF and one or more compounds selected among the group of fetal bovine serum, hydrocortisone, hFGF-B, R3-IGF-1, ascorbic acid, hEGF, GA-1000 and heparin.

23. A method for preparing a vascularized regenerated mammalian tissue, comprising:
a) providing a cell population enriched in vascular stem cells according to any one of claims 15 to 17; and
b) contacting said cell population with an isolated mammalian tissue under conditions suitable for differentiation of the vascular stem cells into vascular cells, thereby obtaining vascularized regenerated mammalian tissue.

24. The method of claim 23, wherein isolated mammalian tissue consists of in vitro cell cultures selected among hepatocytes, epidermal and dermal cells, pancreatic, skeletal muscle tissue, smooth muscle tissue, myocardiac tissue, adipous, cartilaginous and bone tissue, and wherein cell culture is co-cultured with said cell population enriched in vascular stem cells.

25. The method of claim 24, wherein said co-culture is carried out on a 3D matrix or on a semi-porous scaffold.

26. A method for obtaining a cell therapy product comprising
a) providing a cell composition selected from the group of:
i. a population cell enriched in vascular stem cells according to any one of claims 15 to 17;
ii. a vascular tissue as prepared according to any one of claims 18 to 22; and
iii. a vascularized regenerated tissue as prepared according to any one of claims 23 to 25;
b) combining an efficient amount of said cell composition with an appropriate vehicle for *in vivo* administration in a mammal
thereby obtaining a cell therapy product.

27. Use of the cell population enriched in vascular stem cells according to any one of claims 15 to 17, for the preparation of a cell therapy product intended for the treatment in a mammal of post-ischemic cardiac failure or cardiovascular disease.

28. The use according to claim 27, wherein said mammal is a human suffering from post-ischemic cardiac failure or cardiovascular disease.

29. The use according to claim 27, wherein said cell population enriched in vascular stem cells is isolated from an artery of said human suffering from post-ischemic cardiac failure or cardiovascular disease.

30. Use of the cell population enriched in vascular stem cells according to any of claims 15 to 17 for the *in vitro* screening of angiogenic or anti-angiogenic compounds.

31. An *in vitro* method for screening for compounds having angiogenic or anti-angiogenic compound, said method comprising:
a) providing a cell population enriched in vascular stem cells according to any one of claims 15 to 17;
b) combining said cell population with a candidate compound;
c) culturing said cell population under conditions appropriate for growth and differentiation into vascular cells; and
d) determining whether the differentiation of vascular stem cells into vascular cell is enhanced or inhibited, thereby indicating that said candidate compound is respectively an angiogenic or an anti-angiogenic compound respectively.

32. A composition comprising;
a) a cell population enriched in vascular stem cells according to any one of claims 15 to 17; and
b) a physiologically acceptable carrier.

33. A composition as defined in claim 32, for use in revascularizing ischemic or injured, skeletal and myocardial, muscles cells.

34. A kit comprising:
a) a cell population enriched in vascular stem cells according to any one of claims 15 to 17; and
b) reagents necessary for culturing said vascular stem cells.

35. The kit according to claim 34, further comprising an extracellular matrix.

36. The kit according to claim 35, wherein said extracellular matrix is a **Matrigel** basement membrane or collagen gel.

## Patentansprüche

1. Verfahren zum Erhalten einer an vaskulären Stammzellen angereicherten Zellpopulation, umfassend:
a) Bereitstellen einer aus der medialen Tunica und inneren Tunica einer Säugerarterie isolierten Probe;
b) Enzymatisches Verdauen der Probe von Schritt a) zu freien vaskulären Zellen;
c) Kombinieren der in Schritt b) erhaltenen vaskulären Zellen mit einem markierten Vital- und/oder lipophilen Substrat für ATP Binding Cassette-Transporter, unter Bedingungen, die zur Aufnahme des Substrats durch die Zellen geeignet sind;
d) Bestimmen der Menge an in jeder Zelle vorhandenem markiertem Substrat; und
e) Isolieren der Population von nukleierten Zellen, die die geringste Menge des Substrats enthält, dadurch Erhalten einer an vaskulären Stammzellen angereicherten Zellpopulation.

2. Verfahren nach Anspruch 1, wobei das markierte Substrat für ATP Binding Cassette-Transporter ein Vitalfluoreszenzfarbstoff ist.

3. Verfahren nach Anspruch 2, wobei der Schritt d) durchgeführt wird durch Aussetzen der Zellen einer Anregungswellenlänge, die Fluoreszenz des Farbstoffs bewirkt, und Bestimmen der Fluoreszenzemission jeder Zelle.

4. Verfahren nach Anspruch 3, wobei der Farbstoff Hoechst 33342-Farbstoff ist, wobei die Anregungswellenlänge 351 nm beträgt und eine Emissionswellenlänge von 400 bis 700 nm für jede Zelle bestimmt wird.

5. Verfahren nach einem beliebigen der Ansprüche 2 bis 4, wobei Schritte d) und e) unter Verwendung eines FACS-Gerätes durchgeführt werden.

6. Verfahren nach einem beliebigen der Ansprüche 1 bis 5, wobei ein Farbstoff, der Ausschluss von toten Zellen erlaubt, zusätzlich zu dem markierten Substrat hinzugefügt wird.

7. Verfahren nach Anspruch 6, wobei der Farbstoff, der Ausschluss von toten Zellen erlaubt, Propidiumiodid ist.

8. Verfahren nach einem beliebigen der Ansprüche 1 bis 7, wobei die in Schritt a) bereitgestellten Zellen aus einer humanen Arterie isoliert werden.

9. Verfahren nach einem beliebigen der Ansprüche 1 bis 8, wobei die Probe mit Typ II-Collagenase(n) verdaut wird.

10. Verwendung eines gegen Bcrp1/ABCG2 gerichteten Antikörpers zum Detektieren und/oder Reinigen vaskulärer Stammzellen aus Säugerarterienzellen.

11. Verwendung nach Anspruch 10, wobei der Antikörper ein polyklonaler, gegen Bcrp1/ABCG2-Transporter gerichteter Antikörper ist.

12. Verwendung nach Anspruch 10, wobei der Antikörper ein monoklonaler, gegen Bcrp1/ABCG2-Transporter gerichteter Antikörper ist.

13. Verfahren zum Erhalten einer an vaskulären Stammzellen angereicherten Zellpopulation, umfassend:
a) Bereitstellen einer aus der medialen Tunica und inneren Tunica einer Säugerarterie isolierten Probe;
b) Enzymatisches Verdauen der Probe von Schritt a) zu freien vaskulären Zellen; und
c) Auswählen der aus Schritt b) erhaltenen vaskulären Zellen, die einen Bcrp1/ABCG2-Transporter exprimieren.

14. Verfahren nach Anspruch 13, wobei Schritt c) durch FACS-Sortierung durchgeführt wird.

15. Zellpopulation, die an vaskulären Stammzellen angereichert ist, erhältlich durch das Verfahren nach einem beliebigen der Ansprüche 1 bis 9.

16. Zellpopulation nach Anspruch 15, die eine an vaskulären Stammzellen angereicherte humane Zellpopulation ist.

17. Zellpopulation, die an vaskulären Stammzellen angereichert ist, **dadurch gekennzeichnet, dass** sie durch das Verfahren nach Ansprüchen 13 oder 14 erhältlich ist.

18. Verfahren zum Herstellen von vaskulärem Gewebe, umfassend das Kultivieren einer Zeilpopulation, die an vaskulären Stammzellen angereichert ist, gemäß einem beliebigen der Ansprüche 15 bis 17, in der Gegenwart mindestens eines vaskulogenen und/oder angiogenen Wachstumsfaktors, unter Bedingungen, die zum Induzieren von vaskulärer Gewebedifferenzierung geeignet sind, dadurch Erhalten von vaskulärem Gewebe.

19. Verfahren nach Anspruch 18, des Weiteren umfassend eine oder mehrere Expansionsphasen nach Differenzierung des vaskulären Gewebes.

20. Verfahren nach Anspruch 18, wobei der Kulturschritt in einem halbfesten Vaskularisierung fördernden Medium, das Bestandteile extrazellulärer Matrix umfasst, durchgeführt wird.

21. Verfahren nach Anspruch 20, wobei der Bestandteil der extrazellulären Matrix eine Matrigeluntergrundmembran oder ein Kollagengel ist.

22. Verfahren nach Anspruch 20 oder 21, wobei das Medium VEGF und eine oder mehrere Verbindungen, ausgewählt aus der Gruppe von fötalem Rinderserum, Hydrocortison, hFGF-B, R3-IGF-1, Ascorbinsäure, hEGF, GA-1000 und Heparin, umfasst.

23. Verfahren zum Herstellen eines vaskularisierten, regenerierten Säugergewebes, umfassend:
a) Bereitstellen einer an vaskulären Stammzellen angereicherten Zellpopulation gemäß einem beliebigen der Ansprüche 15 bis 17; und
b) In-Kontakt-Bringen der Zellpopulation mit einem isolierten Säugergewebe unter Bedingungen, die zur Differenzierung der vaskulären Stammzellen zu vaskulären Zellen geeignet sind, dadurch Erhalten von vaskularisiertem, regeneriertem Säugergewebe.

24. Verfahren nach Anspruch 23, wobei isoliertes Säugergewebe aus in vitro-Zellkulturen besteht, ausgewählt aus Hepatocyten, epidermalen und dermalen Zellen, Pankreas-, Skelettmuskelgewebe, Gewebe der glatten Muskulatur, Myokardgewebe, Fett-, Knorpel und Knochengewebe, und wobei Zellkultur mit der an vaskulären Stammzellen angereicherten Zellpopulation co-kultiviert wird.

25. Verfahren nach Anspruch 24, wobei die Co-Kultur auf einer 3D-Matrix oder auf einem halbporösen Gerüst durchgeführt wird.

26. Verfahren zum Erhalten eines Zelltherapieprodukts, umfassend
a) Bereitstellen einer Zellzusammensetzung, ausgewählt aus der Gruppe von
i. einer an vaskulären Stammzellen angereicherten Zellpopulation gemäß einem beliebigen der Ansprüche 15 bis 17;
ii. einem vaskulären Gewebe, hergestellt gemäß einem beliebigen der Ansprüche 18 bis 22; und
iii. einem wie in einem beliebigen der Ansprüche 23 bis 25 hergestellten, vaskularisierten, regenerierten Gewebe;
b) Kombinieren einer wirksamen Menge der Zellzusammensetzung mit einem geeigneten Vehikel zur in vivo Verabreichung in einen Säuger;
dadurch Erhalten eines Zelltherapieprodukts.

27. Verwendung der an vaskulären Stammzellen angereicherten Zellpopulation gemäß einem beliebigen der Ansprüche 15 bis 17, zur Herstellung eines Zelltherapieprodukts, das für die Behandlung von post-ischämischem Herzversagen oder Herz-Kreislauf-Erkrankung in einem Säuger beabsichtigt ist.

28. Verwendung nach Anspruch 27, wobei der Säuger ein Mensch ist, der an post-ischämischem Herzversagen oder Herz-Kreislauf-Erkrankung leidet.

29. Verwendung nach Anspruch 27, wobei die an vaskulären Stammzellen angereicherte Zellpopulation aus einer Arterie des Menschen, der an post-ischämischem Herzversagen oder Herz-Kreislauf-Erkrankung leidet, isoliert wird.

30. Verwendung der an vaskulären Stammzellen angereicherten Zellpopulation gemäß einem beliebigen der Ansprüche 15 bis 17 zum in vitro-Screenen von angiogenen oder anti-angiogenen Verbindungen.

31. In vitro-Verfahren zum Screenen auf Verbindungen mit angiogenen oder anti-angiogenen Verbindungen, wobei das Verfahren umfasst:
a) Bereitstellen einer an vaskulären Stammzellen angereicherten Zellpopulation gemäß einem beliebigen der Ansprüche 15 bis 17;
b) Kombinieren der Zellpopulation mit einer Kandidatenverbindung;
c) Kultivieren der Zellpopulation unter Bedingungen, die für Wachstum und Differenzierung in vaskuläre Zellen geeignet sind; und
d) Bestimmen, ob die Differenzierung von vaskulären Stammzellen in vaskuläre Zellen verstärkt oder inhibiert wird, dadurch Indizieren, dass die Kandidatenverbindung eine angiogene bzw. oder eine anti-angiogene Verbindung ist, bzw.

32. Zusammensetzung, umfassend:
a) eine an vaskulären Stammzellen angereicherte Zellpopulation gemäß einem beliebigen der Ansprüche 15 bis 17; und
b) einen physiologisch verträglichen Träger.

33. Zusammensetzung, wie in Anspruch 32 definiert, zur Verwendung in der Revaskulisierung von ischämischen oder verletzten, Skelett- und Herzmuskelzellen.

34. Kit, umfassend:
a) eine an vaskulären Stammzellen angereicherte Zellpopulation gemäß einem beliebigen der Ansprüche 15 bis 17; und
b) zum Kultivieren der vaskulären Stammzellen notwendige Reagenzien.

35. Kit nach Anspruch 34, des Weiteren umfassend eine extrazelluläre Matrix.

36. Kit nach Anspruch 35, wobei die extrazelluläre Matrix eine Matrigeluntergrundmembran oder ein Collagengel ist.

## Revendications

1. Procédé d'obtention d'une population cellulaire enrichie en cellules souches vasculaires, comprenant :
a) la fourniture d'un échantillon isolé à partir de la tunique médiane et de la tunique interne d'une artère de mammifère ;
b) la digestion enzymatique de l'échantillon de l'étape a) pour libérer les cellules vasculaires ;
c) la combinaison des cellules vasculaires obtenues à l'étape (b) avec un substrat vital et/ou lipophile marqué, pour les transporteurs de cassette de liaison à l'ATP, dans des conditions appropriées pour l'absorption du substrat par lesdites cellules ;
d) la détermination de la quantité de substrat marqué présent dans chaque cellule ;
et
e) l'isolement de la population de cellules nucléées, qui contient la plus faible quantité de substrat, pour obtenir ainsi une population cellulaire enrichie en cellules souches vasculaires.

2. Procédé selon la revendication 1, dans lequel ledit substrat marqué pour les transporteurs de cassette de liaison à l'ATP est un colorant vital fluorescent.

3. Procédé selon la revendication 2, dans lequel ladite étape d) est effectuée en exposant les cellules à une longueur d'onde d'excitation qui provoque la fluorescence du colorant et en déterminant l'émission de fluorescence de chaque cellule.

4. Procédé selon la revendication 3, dans lequel ledit colorant est le colorant Hoechst 33342, ladite longueur d'onde d'excitation est 351 nm et une longueur d'onde d'émission de 400 à 700 nm est déterminée pour chaque cellule.

5. Procédé selon l'une quelconque des revendications 2 à 4, dans lequel les étapes d) et e) sont effectuées en utilisant un appareil de FACS.

6. Procédé selon l'une quelconque des revendications 1 à 5, dans lequel un colorant qui permet l'exclusion des cellules mortes est ajouté en plus du substrat marqué.

7. Procédé selon la revendication 6, dans lequel ledit colorant permettant l'exclusion des cellules mortes est l'iodure de propidium.

8. Procédé selon l'une quelconque des revendications 1 à 7, dans lequel lesdites cellules fournies à l'étape (a) sont isolées à partir d'une artère humaine.

9. Procédé selon l'une quelconque des revendications 1 à 8, dans lequel ledit échantillon est digéré par une(des) collagénase(s) de type II.

10. Utilisation d'un anticorps dirigé contre Bcrp1/ABCG2 pour la détection et/ou la purification de cellules souches vasculaires à partir de cellules artérielles de mammifère.

11. Utilisation selon la revendication 10, dans laquelle ledit anticorps est un anticorps polyclonal dirigé contre un transporteur Bcrp1/ABCG2.

12. Utilisation selon la revendication 10, dans laquelle ledit anticorps est un anticorps monoclonal dirigé contre un transporteur Bcrp1/ABCG2.

13. Procédé d'obtention d'une population cellulaire enrichie en cellules souches vasculaires, comprenant :
a) la fourniture d'un échantillon isolé à partir de la tunique médiane et de la tunique interne d'une artère de mammifère ;
b) la digestion enzymatique de l'échantillon de l'étape a) pour libérer les cellules vasculaires ; et
c) la sélection des cellules vasculaires obtenues à partir de l'étape b) qui expriment un transporteur Bcrp1/ABCG2.

14. Procédé selon la revendication 13, dans lequel l'étape c) est effectuée par un tri par FACS.

15. Population cellulaire enrichie en cellules souches vasculaires pouvant être obtenue par le procédé selon l'une quelconque des revendications 1 à 9.

16. Population cellulaire selon la revendication 15 qui est une population de cellules humaines enrichie en cellules souches vasculaires.

17. Population cellulaire enrichie en cellules souches vasculaires, **caractérisée en ce qu'**elle peut être obtenue par le procédé selon les revendications 13 ou 14.

18. Procédé de préparation d'un tissu vasculaire, comprenant la culture d'une population cellulaire enrichie en cellules souches vasculaires selon l'une quelconque des revendications 15 à 17, en présence d'au moins un facteur de croissance vasculogène et/ou angiogénique, dans des conditions appropriées pour induire la différenciation en tissu vasculaire, pour obtenir ainsi un tissu vasculaire.

19. Procédé selon la revendication 18, comprenant en outre une ou plusieurs phases d'expansion, après la différenciation dudit tissu vasculaire.

20. Procédé selon la revendication 18, dans lequel ladite étape de culture est effectuée dans un milieu semi-solide favorisant la vascularisation comprenant des composants de la matrice extracellulaire.

21. Procédé selon la revendication 20, dans lequel ledit composant de la matrice extracellulaire est une membrane basale Matrigel ou un gel de collagène.

22. Procédé selon la revendication 20 ou 21, dans lequel ledit milieu comprend du VEGF et un ou plusieurs composés choisis dans le groupe du sérum bovin foetal, de l'hydrocortisone, du hFGF-B, du R3-IGF-1, de l'acide ascorbique, de l'hEGF, de GA-1000 et de l'héparine.

23. Procédé de préparation d'un tissu de mammifère régénéré vascularisé, comprenant :
a) la fourniture d'une population cellulaire enrichie en cellules souches vasculaires selon l'une quelconque des revendications 15 à 17 ; et
b) la mise en contact de ladite population cellulaire avec un tissu de mammifère isolé dans des conditions appropriées pour la différenciation des cellules souches vasculaires en cellules vasculaires, pour obtenir ainsi un tissu de mammifère régénéré vascularisé.

24. Procédé selon la revendication 23, dans lequel un tissu de mammifère isolé est constitué de cultures cellulaires *in vitro* choisies parmi les hépatocytes, les cellules épidermiques et dermiques, le tissu pancréatique, le tissu des muscles squelettiques, le tissu des muscles lisses, le tissu myocardique, le tissu adipeux, cartilagineux et osseux, et dans lequel la culture cellulaire est cultivée conjointement avec ladite population cellulaire enrichie en cellules souches vasculaires.

25. Procédé selon la revendication 24, dans lequel ladite co-culture est réalisée sur une matrice 3D ou sur un échafaudage semi-poreux.

26. Procédé d'obtention d'un produit de thérapie cellulaire, comprenant
a) la fourniture d'une composition de cellules choisie dans le groupe constitué par :
i. une population cellulaire enrichie en cellules souches vasculaires selon l'une quelconque des revendications 15 à 17 ;
ii. un tissu vasculaire tel que préparé selon l'une quelconque des revendications 18 à 22 ; et
iii. un tissu régénéré vascularisé tel que préparé selon l'une quelconque des revendications 23 à 25 ;
b) la combinaison d'une quantité efficace de ladite composition cellulaire avec un véhicule approprié pour une administration *in vivo* à un mammifère
pour obtenir ainsi un produit de thérapie cellulaire.

27. Utilisation de la population cellulaire enrichie en cellules souches vasculaires selon l'une quelconque des revendications 15 à 17, pour la préparation d'un produit de thérapie cellulaire destiné au traitement chez un mammifère d'une insuffisance cardiaque post-ischémique ou d'une maladie cardio-vasculaire.

28. Utilisation selon la revendication 27, dans laquelle ledit mammifère est un humain souffrant d'une insuffisance cardiaque post-ischémique ou d'une maladie cardio-vasculaire.

29. Utilisation selon la revendication 27, dans laquelle ladite population cellulaire enrichie en cellules souches vasculaires est isolée à partir d'une artère dudit humain souffrant d'une insuffisance cardiaque post-ischémique ou d'une maladie cardio-vasculaire.

30. Utilisation de la population cellulaire enrichie en cellules souches vasculaires selon l'une quelconque des revendications 15 à 17, pour le criblage *in vitro* des composés angiogéniques ou anti-angiogéniques.

31. Procédé *in vitro* pour le criblage des composés ayant un composé angiogénique ou anti-angiogénique, ledit procédé comprenant :
a) la fourniture d'une population cellulaire enrichie en cellules souches vasculaires selon l'une quelconque des revendications 15 à 17 ;
b) la combinaison de ladite population cellulaire avec un composé candidat ;
c) la culture de ladite population cellulaire dans des conditions appropriées pour la croissance et la différenciation en cellules vasculaires ; et
d) la détermination si la différenciation des cellules souches vasculaires en cellules vasculaires est activée ou inhibée, indiquant ainsi que ledit composé candidat est respectivement un composé angiogénique ou un composé anti-angiogénique, respectivement.

32. Composition comprenant :
a) une population cellulaire enrichie en cellules souches vasculaires selon l'une quelconque des revendications 15 à 17 ; et
b) un support physiologiquement acceptable.

33. Composition selon la revendication 32, destinée à une utilisation dans la revascularisation de cellules des muscles squelettiques et myocardiques, ischémiques ou lésés.

34. Trousse comprenant :
a) une population cellulaire enrichie en cellules souches vasculaires selon l'une quelconque des revendications 15 à 17 ; et
b) les réactifs nécessaires à la culture desdites cellules souches vasculaires.

35. Trousse selon la revendication 34, comprenant en outre une matrice extracellulaire.

36. Trousse selon la revendication 35, dans laquelle ladite matrice extracellulaire est une membrane basale Matrigel ou un gel de collagène.
